# EUROPEAN PATENT APPLICATION

(11) **EP 2 843 006 A1**
(43) Date of publication of application: **04.03.2015**
(21) Application number: 13781697.1
(22) Date of filing: 08.04.2013
(51) Int. Cl.: C09C 3/10, A61K 8/25, A61K 8/26, A61K 8/27, A61K 8/28, A61K 8/29, A61Q 17/04, C01G 23/04, C09C 1/04, C09C 1/28, C09C 1/36, C09C 1/40

(54) **SURFACE MODIFIED INORGANIC OXIDE FINE PARTICLES, AND SUNSCREEN COSMETIC MATERIAL CONTAINING SAME**

(30) Priority: 23.04.2012 JP 2012097269
(71) Applicant: Daicel Corporation, Osaka-shi, Osaka 530-0001 (JP)
(72) Inventor: SAKANISHI, Yuichi, Ohtake-shi Hiroshima 739-0695 (JP)
(74) Representative: Grünecker, Kinkeldey, Stockmair & Schwanhäusser
(86) International application number: PCT/JP2013/060612
(87) International publication number: WO 2013/161553

(57) **Abstract**

Provided are surface-modified inorganic oxide fine particles that are added with water dispersibility while maintaining properties of inorganic oxide fine particles, such as transparency, ultraviolet blocking ability, weatherability, and photodiscoloration resistance; a method for producing the surface-modified inorganic oxide fine particles; and a sunscreen cosmetic containing the surface-modified inorganic oxide fine particles.

The surface-modified inorganic oxide fine particles include inorganic oxide fine particles having an average particle size of 200 nm or less; and a group containing a polyglycerol chain and modifying surfaces of the particles. The inorganic oxide is preferably selected from titanium oxides, silicon oxides, aluminum oxides, zirconium oxide, and zinc oxide.

## Description

### Technical Field

The present invention relates to surface-modified inorganic oxide fine particles that are added with water dispersibility while maintaining properties of inorganic oxide fine particles, such as transparency, ultraviolet blocking ability, weatherability, and resistance to color change by light (photodiscoloration resistance); and to sunscreen cosmetics containing the particles.

### Background Art

Inorganic oxides such as titanium oxides are known to exhibit properties that vary depending on their particle sizes. Typically, fine particles having an average particle size of 200 nm or less offer better transparency and ultraviolet blocking ability than pigment grade particles having an average particle size greater than 200 nm (0.2 µm). The fine particles are used in the fields typically of coating materials, sunscreen cosmetics, and resin-use compositions while taking advantage of the properties.

Coating of such fine particles typically of titanium oxide with inorganic compounds of various kinds improves their property or properties, or adds a new property to them. For example, titanium oxide fine particles have smaller particle sizes, larger specific surface areas, and higher photoactivity than pigment grade titanium oxide particles. The titanium oxide fine particles are therefore inferior in weatherability and photodiscoloration resistance to the pigment grade titanium oxide particles. As a possible solution to this, there has been proposed a technique of imparting weatherability and photodiscoloration resistance to titanium oxide fine particles by coating the surfaces of the particles with zirconium oxide, hydrous zirconium oxide, aluminum oxide, and hydrous aluminum oxide (Patent Literature (PTL) 1 and PTL 2). The technique is used in the fields typically of coating materials.

Independently, there has been proposed a technique of helping titanium oxide fine particles to have better transparency and/or ultraviolet blocking ability by coating surfaces of the particles with silicon oxide and aluminum oxide (PTL 3 and PTL 4). The technique is used in the fields typically of packaging materials, coating materials, and inks.

There are recently increasing moves afoot to produce products including water, instead of organic solvents, as a dispersion medium. The moves occur in many fields, as giving consideration to environmental friendliness. Such water-borne compositions are more and more used in the above-mentioned fields. The titanium oxide fine particles and other inorganic oxide fine particles themselves and compounds obtained by coating their surfaces with an inorganic compound offer hydrophobicity and have excellent dispersibility when an organic solvent is used as a dispersion medium. Unfortunately, however, they are liable to aggregate when compounded into water-borne coating materials.

### Citation List

### Patent Literature

PTL 1: Japanese Examined Patent Application Publication (JP-B) H07-751
PTL 2: Japanese Unexamined Patent Application Publication (JP-A) No. H04-81470
PTL 3: JP-A No. S55-10428
PTL 4: JP-A No. S55-154317

### Summary of Invention

### Technical Problem

Accordingly, an object of the present invention is to provide surface-modified inorganic oxide fine particles that are added with water dispersibility while maintaining properties of inorganic oxide fine particles, such as transparency, ultraviolet blocking ability, weatherability, and photodiscoloration resistance.

Another object of the present invention is to provide a method for producing the surface-modified inorganic oxide fine particles.

Yet another object of the present invention is to provide a sunscreen cosmetic containing the surface-modified inorganic oxide fine particles.

### Solution to Problem

After intensive investigations to achieve the objects, the inventor has found that surface modification of inorganic oxide fine particles by binding a chemical group present in surfaces of the particles to a polyglycerol chain can give modified inorganic oxide fine particles having significantly better dispersibility in water and a polar organic solvent; and that the resulting surface-modified inorganic oxide fine particles still maintain, without deterioration, properties of the inorganic oxide fine particles before modification, such as transparency, ultraviolet blocking ability, weatherability, and photodiscoloration resistance. The present invention has been made based on these findings.

Specifically, the present invention provides surface-modified inorganic oxide fine particles including inorganic oxide fine particles having an average particle size of 200 nm or less; and a group containing a polyglycerol chain and modifying surfaces of the inorganic oxide fine particles.

The inorganic oxide is preferably selected from titanium oxides, silicon oxides, aluminum oxides, zirconium oxide, and zinc oxide.

The present invention further provides a sunscreen cosmetic containing the surface-modified inorganic oxide fine particles.

In addition and advantageously, the present invention provides a method for producing surface-modified inorganic oxide fine particles, comprising the step of subjecting inorganic oxide fine particles to ring-opening addition polymerization with glycidol, the inorganic oxide fine particles each having, in a surface thereof, a chemical group reactive with glycidol.

### Advantageous Effects of Invention

The surface-modified inorganic oxide fine particles according to the present invention has the configuration and are added with excellent dispersibility and dispersion stability in water and a polar organic solvent without impairing properties of the inorganic oxide fine particles before modification, such as transparency, ultraviolet blocking ability, weatherability, and photodiscoloration resistance. The surface-modified inorganic oxide fine particles can be kept in a stable dispersion state without aggregation even when dispersed in water as a dispersion medium. The surface-modified inorganic oxide fine particles according to the present invention are advantageously usable typically in environmentally friendly water-borne coating materials, sunscreen cosmetics, and compositions to form resins.

### Description of Embodiments

### Surface-modified Inorganic Oxide Fine Particles

The surface-modified inorganic oxide fine particles according to an embodiment of the present invention include inorganic oxide fine particles having an average particle size of 200 nm or less; and a group containing a polyglycerol chain and modifying surfaces of the inorganic oxide fine particles.

Inorganic oxide fine particles are known to have various chemical groups in their surfaces. Inorganic oxide fine particles for use in the present invention as a material preferably have, among such chemical groups, a chemical group reactive with glycidol (hereinafter briefly referred to as "glycidol-reactive chemical group"). Specifically, the surface-modified inorganic oxide fine particles according to the present invention are preferably modified with a polyglycerol chain that is bonded to surfaces of inorganic oxide fine particles via the glycidol-reactive chemical group.

The glycidol-reactive chemical group is exemplified by -NH₂, -OH, -COOH, -P(=O)-OH, and -SH groups. The inorganic oxide fine particles for use herein may have each of chemical groups alone or in combination. Among them, the inorganic oxide fine particles preferably have an -OH group.

The inorganic oxide fine particles are exemplified by fine particles of silicon oxides, titanium oxides, aluminum oxides, zirconium oxide, and zinc oxide. Among them, preferred herein are titanium oxides and zinc oxide, of which titanium oxides (rutile and anatase titanium dioxides) are most preferred. This is because they have excellent ultraviolet blocking ability.

The silicon oxides, titanium oxides, aluminum oxides, zirconium oxide, and zinc oxide may each be coated with another inorganic compound on the surface. Specifically, the silicon oxides, titanium oxides, aluminum oxides, zirconium oxide, and zinc oxide may be those coated with another inorganic compound so as to have a better property or to be added with another property.

The inorganic compound to cover the surfaces of titanium oxide fine particles is exemplified by inorganic oxides such as Al₂O₃, SiO₂, ZrO₂, SnO₂, and Sb₂O₃; and hydrous inorganic oxides corresponding to the inorganic oxides. Compounds obtained by coating the surfaces of titanium oxide fine particles with Al₂O₃ and/or SiO₂ offer still better transparency and ultraviolet blocking ability than the titanium oxide fine particles before coating. Compounds obtained by coating the surfaces of the titanium oxide fine particles with ZrO₂, hydrous ZrO₂, Al₂O₃, and hydrous Al₂O₃ have weatherability and photodiscoloration resistance in addition to transparency and ultraviolet blocking ability. Compounds obtained by coating the surfaces of the titanium oxide fine particles with SnO₂ and/or Sb₂O₃ have electroconductivity in addition to transparency and ultraviolet blocking ability.

The inorganic compound to cover the surfaces of zinc oxide fine particles is exemplified by inorganic oxides such as SiO₂ and Al₂O₃. Compounds obtained by coating the surfaces of the zinc oxide fine particles with SiO₂ or Al₂O₃ have still better transparency and ultraviolet blocking ability than the zinc oxide fine particles before coating.

The inorganic oxide fine particles have an average particle size of 200 nm or less. The upper limit of the average particle size (volume-average diameter) of the inorganic oxide fine particles is preferably 150 nm, and more preferably 100 nm. The lower limit thereof is preferably 1 nm, more preferably 3 nm, furthermore preferably 5 nm, particularly preferably 7 nm, and most preferably 10 nm. The average particle size of the inorganic oxide fine particles may be measured by the Nanotrac method (i.e., dynamic light scattering method). When the inorganic oxide fine particles are fusiform, the term "average particle size" refers to an average length of major axis. Inorganic oxide fine particles, when having an average particle size within the range, offer excellent transparency and ultraviolet blocking ability.

The chemical group and the average particle size of the inorganic oxide fine particles may be controlled typically by adjusting their production method, producing conditions, and classification operation conditions after production. The inorganic oxide fine particles for use herein may be preferably available as a commercial product typically under the trade name of STR-100A from Sakai Chemical Industry Co., Ltd. This product is fusiform rutile titanium dioxide fine particles that are coated with SiO2/Al₂O₃ and have an average length of major axis of 75 nm and an average length of minor axis of 15 nm. Such commercially available inorganic oxide fine particles may be further subjected to chemical modification before the reaction with glycidol.

The polyglycerol chain may have a number-average polymerization degree not critical, as long as within such a range as to give target high dispersibility, but typically from 2 to 100. The upper limit of the number-average polymerization degree of the polyglycerol chain is preferably 40, more preferably 20, and most preferably 15; and the lower limit thereof is preferably 3, and particularly preferably 5. The polyglycerol chain, if having an excessively small number-average polymerization degree, may readily cause the inorganic oxide fine particles to have insufficient repulsive force from one another and may fail to help the inorganic oxide fine particles to resist aggregation and to be kept in a stable dispersion state in water. In contrast, the polyglycerol chain, if having an excessively large number-average polymerization degree, may readily cause the surface-modified inorganic oxide fine particles to aggregate, because the polyglycerol chains of the respective particles may be entangled with one another. In addition, such polyglycerol chain having an excessively large number-average polymerization degree may readily dilute the properties of the inorganic oxide fine particles, such as transparency, ultraviolet blocking ability, weatherability, and photodiscoloration resistance. As used herein the term "number-average polymerization degree" is defined by the number of glycerol units constituting a polyglycerol chain bonded to a surface functional group of the inorganic oxide fine particles.

The amount of the polyglycerol chain to be introduced is not critical, as long as within such a range as to offer high dispersibility in water, but may be such an amount that the weight of a chemical modification moiety including the polyglycerol chain is typically from about 4 to about 750 parts by weight, per 100 parts by weight of the weight of the inorganic oxide fine particle moiety. The upper limit of the weight of the chemical modification moiety including the polyglycerol chain is preferably 380 parts by weight, and particularly preferably 150 parts by weight; and the lower limit thereof is preferably 9 parts by weight, particularly preferably 13 parts by weight, more preferably 50 parts by weight, and most preferably 70 parts by weight, per 100 parts by weight of the weight of the inorganic oxide fine particle moiety. The chemical modification moiety including the polyglycerol chain, if introduced in an excessively small amount, may fail to cover the inorganic oxide fine particle surface sufficiently and may fail to help the inorganic oxide fine particles to resist aggregation and to be kept in a stable dispersion state in water. In contrast, the chemical modification moiety including the polyglycerol chain, if introduced in an excessively large amount, may often dilute the inherent properties of the inorganic oxide fine particles, such as transparency, ultraviolet blocking ability, weatherability, and photodiscoloration resistance. The weight ratio of the surface-introduced chemical modification moiety including the polyglycerol chain to the inorganic oxide fine particle moiety may be determined typically by measuring a weight change of the surface-modified inorganic oxide fine particles upon a heat treatment with a differential thermogravimetric analyzer (TG-DTA); or measuring a compositional ratio by elementary analysis.

### Manufacturing Method of Surface-modified Inorganic Oxide Fine Particles

The surface-modified inorganic oxide fine particles according to the present invention may be produced by subjecting inorganic oxide fine particles to ring-opening addition polymerization with glycidol, where the inorganic oxide fine particles have a glycidol-reactive chemical group in their surfaces.

For glycidol ring-opening polymerization conditions, reference may be made typically to S. R Sandler et al., J. Polym. Sci., Polym. Chem. Ed., Vol. 4, 1253(1966); E. J. Vanderberg, J. Polym. Sci., Polym. Chem. Ed., vol. 23, 915(1985); and G. R. Newcome et al., "Dendritic Macromolecules: Concepts, Syntheses, Perspectives," VCH, Weinheim (1996), as appropriate.

The ring-opening addition polymerization between glycidol and the inorganic oxide fine particles having a glycidol-reactive chemical group in surface may be performed in the presence of, or in the absence of, a catalyst. In the former case, exemplary catalysts usable herein include acidic catalysts such as boron trifluoride etherate, acetic acid, and phosphoric acid; and basic catalysts such as triethylamine, pyridine, dimethylaminopyridine, and triphenylphosphine.

The catalyst may be used in an amount of typically from about 0.01 to about 0.1 part by weight and preferably from 0.02 to 0.05 part by weight, per 100 parts by weight of the inorganic oxide fine particles.

The ring-opening addition polymerization may be performed at a temperature of typically from about 50°C to about 180°C, preferably from 80°C to 150°C, and particularly preferably from 100°C to 150°C; for a reaction time of typically from about 0.5 to about 48 hours, preferably from 5 to 48 hours, and particularly preferably from 10 to 30 hours.

The ring-opening addition polymerization may be performed under normal atmospheric pressure, under reduced pressure, or under pressure (under a load). The ring-opening addition polymerization may be performed in any reaction atmosphere not critical, as long as not adversely affecting the reaction, and may be selected from any atmospheres such as air, nitrogen, and argon atmospheres.

After the completion of the reaction, a product may be purified by a separation-purification process such as concentration, precipitation, centrifugal separation, filtration, extraction, washing, or drying, or any combination of them.

The surface-modified inorganic oxide fine particles obtained by the method maintain the properties of the inorganic oxide fine particles, such as transparency, ultraviolet blocking ability, weatherability, and photodiscoloration resistance and still excel in dispersibility and dispersion stability in water and a polar organic solvent. The surface-modified inorganic oxide fine particles are therefore usable not only in cosmetics and similar fields, but also in engineering application fields such as abrasives and dresser materials for CMP. Above all, they are preferably used as a sunscreen cosmetic. The surface-modified inorganic oxide fine particles according to the present invention, when dispersed in water, give a sunscreen cosmetic. The sunscreen cosmetic has a fresh and dewy feeling, spreads well, and can be easily washed away with water.

### Sunscreen Cosmetic

The sunscreen cosmetic according to an embodiment of the present invention contains the surface-modified inorganic oxide fine particles. As used herein the term "sunscreen cosmetic" refers to a cosmetic having a protection action against ultraviolet light. Such cosmetics include skin-care cosmetics such as lotions, milky lotions, creams, and beauty essence; makeup cosmetics such as lip gloss, lipsticks, mascara, foundation, eyeshadows, eyeliners, eyebrow pencils or paints, and cheek color cosmetics; and sunblock agents (sunblock cosmetics) in the form of cream, emulsion, or gel.

The sunscreen cosmetic may contain the surface-modified inorganic oxide fine particles in a content typically from about 0.1 to about 30 percent by weight and preferably from 1 to 20 percent by weight, based on the total amount (100 percent by weight) of the sunscreen cosmetic.

The sunscreen cosmetic according to the present invention may be prepared by adding the surface-modified inorganic oxide fine particles to one or more cosmetic materials.

The cosmetic materials are exemplified by components for use in regular cosmetics, including water; surfactants; viscosity modifiers; moisturizing components such as polyols; oily components such as squalane, jojoba oil, olive oil, higher alcohols, lanolin, esters, and silicones; amides; foam increasing agents; antiseptic agents; watersoluble polymers; pH adjusters; pearling agents; antioxidants; flavors; and coloring agents. They may be appropriately compounded within ranges not adversely affecting advantageous effects of the present invention.

The sunscreen cosmetic according to the present invention contains the surface-modified inorganic oxide fine particles and thereby offers an excellent protection action against ultraviolet light. The sunscreen cosmetic also offers excellent dispersion stability, has transparency, and, even when applied to the skin, can be finished naturally without a powdery finish.

### Examples

The present invention will be illustrated in further detail with reference to several examples below. It should be noted, however, that the examples are by no means intended to limit the scope of the invention.

### Example 1: Manufacturing of titanium oxide fine particles introduced with polyglycerol chain

Material titanium oxide fine particles were prepared as a commercial product available under the trade name of STR-100A from Sakai Chemical Industry Co., Ltd. The product was fusiform rutile titanium dioxide coated with SiO₂/Al₂O₃ and had an average length of major axis of 75 nm and an average length of minor axis of 15 nm. The material particles were dried at 50°C at a highly reduced pressure of 0.09 mmHg for 30 minutes before a reaction.

The dried titanium oxide fine particles (0.05 g) were placed in a glass reactor, successively combined with 8 g of glycidol, and subjected to a ultrasonic treatment in an ultrasonic cleaner (45 kHz) at room temperature for 30 minutes to form a homogeneous dispersion, followed by stirring at 140°C in a nitrogen atmosphere for 20 hours.

The reaction mixture was combined with methanol and subjected to centrifugal separation at 5000 rpm, 20°C for 90 minutes, from which the supernatant was removed. The procedure including addition, stirring, and dispersion of methanol to (in) the precipitate and centrifugal separation (at 5000 rpm, 20°C for 60 minutes) was repeated twice; the supernatant liquid was removed; the resulting precipitate was combined with methanol, stirred, and dispersed therein, subjected to a fourth centrifugal separation (at 5000 rpm, 20°C for 60 minutes), dried, and yielded a yellow precipitate.

The resulting precipitate was subjected to diffuse reflectance infrared spectrometry to find that a peak component assigned to hydroxyl group of the polyglycerol chain appeared at around 3350 cm⁻¹; and a peak component assigned to ether bond of the polyglycerol chain appeared at around 1095 cm⁻¹.

The precipitate was also subjected to TG-DTA to find that the polyglycerol chain was introduced in an amount of 88 percent by weight relative to the total amount of the material titanium oxide fine particles; and that the polyglycerol chain had an average polymerization degree of 8 (the polyglycerol chain was an octamer on average).

### Evaluations

The polyglycerol-chain-introduced titanium oxide fine particles obtained in the example were examined to evaluate water dispersibility and UV-visible blocking ability by methods as follows.

### Water Dispersion Test and Average Particle Size Measurement

Each 10.0 mg of the polyglycerol-chain-introduced titanium oxide fine particles obtained in the example and the titanium oxide fine particles (trade name STR-100A, having an average particle size of 16 nm, supplied by Sakai Chemical Industry Co., Ltd.) used as the material in the example were respectively placed in vials, combined with 1 mL of pure water, and irradiated with ultrasound for 2 minutes, followed by appearance observation and particle size and its distribution measurement.

Results thereof demonstrate that introduction of the polyglycerol chain helped the inorganic oxide fine particles to have significantly better dispersibility.

The results are collectively indicated in a table as follows.
[Table 1]

**TABLE 1**

| | Material titanium oxide fine particles | Polyglycerol-chain-introduced titanium oxide fine particles |
|---|---|---|
| Appearance | completely separated | semitransparent |
| Average particles size (nm) | unmeasurable due to aggregation | 220 |

### UV-visible Blocking Ability Test

In a screw-capped tube, 50.0 mg of the polyglycerol-chain-introduced titanium oxide fine particles obtained in the example were placed, dispersed in water, and yielded an aqueous solution in a total amount of 10 g.

The total luminous transmittance of the aqueous solution was measured with the UV-VIS Spectrophotometer (trade name UV-2450, supplied by Shimadzu Corporation).

Results thereof demonstrate that almost no light at wavelengths of 380 nm or less in the ultraviolet region was transmitted through the polyglycerol-chain-introduced titanium oxide fine particles.

### Industrial Applicability

The surface-modified inorganic oxide fine particles according to the present invention are added with excellent dispersibility and dispersion stability in water and a polar organic solvent without impairing the properties of the inorganic oxide fine particles before modification, such as transparency, ultraviolet blocking ability, weatherability, and photodiscoloration resistance. The surface-modified inorganic oxide fine particles can thereby be kept in a stable dispersion state without aggregation even when dispersed in water as a dispersion medium and are advantageously usable in environmentally friendly water-borne coating materials, sunscreen cosmetic, and compositions to form resins.

## Claims

1. Surface-modified inorganic oxide fine particles comprising:
inorganic oxide fine particles having an average particle size of 200 nm or less; and
a group comprising a polyglycerol chain and modifying surfaces of the inorganic oxide fine particles.

2. The surface-modified inorganic oxide fine particles according to claim 1,
wherein the inorganic oxide is selected from the group consisting of titanium oxides, silicon oxides, aluminum oxides, zirconium oxide, and zinc oxide.

3. A sunscreen cosmetic comprising the surface-modified inorganic oxide fine particles of one of claims 1 and 2.

4. A method for producing surface-modified inorganic oxide fine particles, comprising the step of:
subjecting inorganic oxide fine particles to ring-opening addition polymerization with glycidol, the inorganic oxide fine particles each comprising, on a surface, a chemical group reactive with glycidol.
